# EUROPÄISCHE PATENTSCHRIFT

(11) **EP 3 878 194 B1**
(45) Veröffentlichungstag und Bekanntmachung des Hinweises auf die Patenterteilung: **09.09.2026**
(21) Anmeldenummer: 20739632.6
(22) Anmeldetag: 09.07.2020
(51) Int. Cl.: H04L 9/40, A61B 6/00, H04W 12/10, H04W 12/50

(54) **VERSORGUNGSSYSTEM UND VERFAHREN ZUR VERSORGUNG EINES MEDIZINISCHEN GERÄTS MIT EINEM FLUID, UMFASSEND EINE DRAHTLOSE BETÄTIGUNGSEINHEIT**
SUPPLY SYSTEM AND METHOD FOR SUPPLYING A MEDICAL DEVICE WITH A FLUID, COMPRISING A WIRELESS ACTUATION UNIT
SYSTÈME D'ALIMENTATION ET PROCÉDÉ POUR ALIMENTER UN APPAREIL MÉDICAL EN FLUIDE, COMPRENANT UNE UNITÉ D'ACTIONNEMENT SANS FIL

(30) Priorität: 19.07.2019 DE 102019005014
(43) Veröffentlichungstag der Anmeldung: 15.09.2021
(73) Patentinhaber: Drägerwerk AG & Co. KGaA, 23558 Lübeck (DE)
(72) Erfinder: BEHLER, Maximilian, 23558 Lübeck (DE); SIEVERS, Falko, 23558 Lübeck (DE); KAHNS, Peter, 23558 Lübeck (DE)
(86) Internationale Anmeldenummer: PCT/EP2020/069383
(87) Internationale Veröffentlichungsnummer: WO 2021/013564

(56) Entgegenhaltungen:
- WO-A1-2006/114297
- DE-A1- 102016 015 368
- US-A1- 2014 281 547

## Beschreibung

Die Erfindung betrifft ein Versorgungssystem und ein Verfahren, welche ein medizinisches Gerät mit mindestens einem Fluid zu versorgen vermögen. Das medizinische Gerät ist beispielsweise ein Beatmungsgerät oder ein Anästhesiegerät oder ein Patienten-Monitor. Das Fluid ist beispielsweise Atemluft oder reiner Sauerstoff oder Lachgas (N2O) oder Wasser oder eine Reinigungsflüssigkeit oder eine desinfizierende Flüssigkeit.

Verschiedene derartige Versorgungssysteme sind bekannt geworden.

In DE 102016015368 wird eine medizinische Vorrichtung beschrieben, welche einen Akkumulator zum physiologischen Einwirken auf einen an die Vorrichtung angeschlossenen Patienten umfasst. Ferner umfasst diese Vorrichtung eine Netzwerkschnittstelle. Die Netzwerkschnittstelle ist ausgebildet, wenigstens eine Datennachricht zu empfangen, welche eine Netzwerkidentität eines Absenders der Nachricht, wenigstens eine Berechtigungsstufe des Absenders sowie eine Anforderung des Absenders zum Verändern eines Betriebsparameters des Aktuators indiziert.

In DE 10 2017 005 011 B3 wird eine Überwachungseinrichtung beschrieben, welche eine Anlage zur Erzeugung medizinischer Druckluft überwacht. Druckluft wird über eine Messleitung 3 abgesogen. Ein Sensor 2 misst eine Eigenschaft der abgesogenen Druckluft. Ein Luftbefeuchter 8 befeuchtet die Druckluft.

In DE 10 2014 018 727 A1 wird ein Versorgungssystem beschrieben, bei welchem eine Quelle 1 wenigstens zwei Verbraucher 2 über ein Versorgungsnetz 3 mit mindestens einem Fluid oder mit elektrischer Energie versorgt. Mittels eines Steckers 7 und einer Kupplung 6 lässt sich ein Steckverbinder 5 herstellen, durch den ein Fluid zu einem Verbraucher 2 fließt. Eine mechanische Codierung 8 stellt sicher, dass die richtigen Stecker 7 und Kupplungen 6 miteinander verbunden werden.

Gewünscht wird, dass ein Benutzer die Zufuhr des Fluids zum medizinischen Gerät verändern kann.

Verschiedene Betätigungseinheiten für medizinische Geräte sind bekannt geworden.

In DE 10 2017 220 532 A1 wird eine als Röntgeneinrichtung ausgebildete Bildaufnahmeeinrichtung 2 mit mehreren bewegbaren Komponenten 3 sowie einer Steuereinrichtung 4 beschrieben. Ein als Smartphone ausgebildetes Smartdevice 5 vermag Eingaben eines Benutzers zu erfassen. Ein Funkinterface der Steuereinrichtung 6 des Smartphones 5 vermag über eine drahtlose Kommunikationsverbindung 7 an die Steuereinrichtung 4 drahtlos Nachrichten zu übermitteln. Mithilfe des Smartphones 5 wählt ein Benutzer eine Komponente 3 der Röntgeneinrichtung 2 aus und veranlasst, dass ein Stellglied diese ausgewählte Komponente 3 bewegt.

In DE 20 2009 012 808 U1 wird ein Pulssensor 10 beschrieben, der den Puls eines Patienten misst und einen Hauptkörper 11, eine Batterie 15 und einen Ladeanschluss 16 umfasst. Der Pulssensor 10 hat beispielsweise die Form eines Rings 10a, der auf einen Finger des Patienten gesteckt werden kann, oder eines Sensors 10b, der auf die Brust des Patienten geklebt werden kann.

Der Erfindung liegt die Aufgabe zugrunde, ein Versorgungssystem und ein Verfahren zur Versorgung eines medizinischen Geräts mit mindestens einem Fluid bereitzustellen, bei welchen die Bereitstellung des Fluids sich leichter als bei bekannten Versorgungssystemen verändern lässt.

Die Aufgabe wird durch ein Versorgungssystem mit den Merkmalen des Anspruchs 1 und durch ein Verfahren mit den Merkmalen des Anspruchs 10 gelöst. Vorteilhafte Ausgestaltungen sind in den Unteransprüchen angegeben. Vorteilhafte Ausgestaltungen des erfindungsgemäßen Versorgungssystems sind auch vorteilhafte Ausgestaltungen des erfindungsgemäßen Verfahrens und umgekehrt.

Das erfindungsgemäße Versorgungssystem und das erfindungsgemäße Verfahren sind dazu ausgestaltet, ein medizinisches Gerät mit mindestens einem Fluid zu versorgen. Das medizinische Gerät ist beispielsweise ein Beatmungsgerät oder ein Anästhesiegerät oder ein Patienten-Monitor oder auch eine Atemmaske. Das oder jedes bereitgestellte Fluid kann gasförmig oder flüssig sein. Ein gasförmiges Fluid kann relativ zum Umgebungsdruck unter einem Unterdruck oder unter einem Überdruck stehen. Das oder ein gasförmiges Fluid ist beispielsweise Atemluft, Sauerstoff, Lachgas (N2O) oder auch ein Anästhesiemittel. Die Flüssigkeit kann Wasser oder eine sonstige Reinigungsflüssigkeit sein oder umfassen.

Das Versorgungssystem umfasst eine Versorgungseinheit und eine Betätigungseinheit.

Die Versorgungseinheit umfasst mindestens einen Fluid-Versorgungsanschluss, bevorzugt mehrere Fluid-Versorgungsanschlüsse. Der oder jeder Fluid-Versorgungsanschluss ist dazu ausgestaltet, jeweils ein Fluid für ein medizinisches Gerät bereitzustellen.

Eine Betätigungseinheit des Versorgungssystems umfasst mindestens ein Betätigungselement sowie einen Sender. Das oder jedes Betätigungselement der Betätigungseinheit lässt sich von einem Menschen betätigen. Optional umfasst das Versorgungssystem mehrere Betätigungseinheiten mit jeweils mindestens einem Betätigungselement und einem Sender.

Die Versorgungseinheit umfasst einen Empfänger. Zwischen dem jeweiligen Sender einer Betätigungseinheit und dem Empfänger der Versorgungseinheit lässt sich wenigstens zeitweise eine drahtlose Datenverbindung herstellen, insbesondere mittels elektromagnetischer Wellen. Wenn eine Datenverbindung zwischen dem Sender der oder einer Betätigungseinheit und dem Empfänger der Versorgungseinheit hergestellt ist, vermag dieser Sender eine Nachricht drahtlos an den Empfänger zu übermitteln.

Die oder jede Betätigungseinheit vermag eine Betätigung des oder eines Betätigungselements dieser Betätigungseinheit zu erfassen. Weiterhin vermag die Betätigungseinheit eine Nachricht zu erzeugen, wobei diese Nachricht eine Betätigungsinformation umfasst und wobei diese Betätigungsinformation die erfasste Betätigung des Betätigungselements kennzeichnet. Dann, wenn eine Datenverbindung zu dem Empfänger der Versorgungseinheit hergestellt ist, vermag der Sender der Betätigungseinheit eine Nachricht mit der erzeugten Betätigungsinformation über diese Datenverbindung an den Empfänger der Versorgungseinheit zu übermitteln. Dadurch wird eine Kennzeichnung der erfassten Betätigung drahtlos an die Versorgungseinheit übermittelt.

Die Versorgungseinheit vermag einen Parameter des oder mindestens eines, bevorzugt jedes Fluid-Versorgungsanschlusses, zu verändern, d.h. den Wert dieses Parameters zu verändern. Im Folgenden wird die Veränderung eines Parameters des oder eines Fluid-Versorgungsanschlusses als "eine Betätigung des Fluid-Versorgungsanschlusses" bezeichnet. Diese Veränderung des Parameters, also die Betätigung, führt die Versorgungseinheit automatisch als Reaktion auf eine empfangene Nachricht mit einer Betätigungsinformation und abhängig von der empfangenen Betätigungsinformation durch.

Der veränderte Parameter beeinflusst die Bereitstellung von Fluid und ist insbesondere
- eine Flussrate, also ein Maß für die Menge pro Zeiteinheit,
- der Druck, bevorzugt ein Druck relativ zum Umgebungsdruck,
- eine Temperatur oder
- eine chemische Zusammensetzung
des bereitgestellten Fluids. Die Veränderung des Parameters kann auch ein Inkrement sein, also eine Veränderung der Flussrate, des Drucks oder der Temperatur.

Der oder ein Parameter kann auch der Beginn und / oder die Dauer einer Zeitspanne, während der dieses Fluid bereitgestellt werden soll, sein, wobei die Bereitstellung nach Ablauf dieser Zeitspanne automatisch beendet werden soll. Die Veränderung des Parameters kann auch darin bestehen, dass die Bereitstellung des Fluids begonnen (Flussrate größer null) oder beendet (Flussrate gleich null) wird.

Die erfindungsgemäße Betätigungseinheit ermöglicht es, die Versorgungseinheit zu betätigen. Dank der Erfindung ist es nicht erforderlich, direkt das medizinische Gerät zu betätigen, um die Zufuhr von Fluid zu dem medizinischen Gerät zu verändern. Das medizinische Gerät lässt sich daher aus der Ferne betätigen. Diese Wirkung der Erfindung reduziert außerdem in vielen Fällen die Varianz unter Betätigungseinheiten, weil es häufig wesentlich weniger unterschiedliche Versorgungseinheiten als zu versorgende medizinische Geräte gibt. In vielen Fällen vermag dieselbe Versorgungseinheit nacheinander unterschiedliche medizinische Geräte zu versorgen.

Erfindungsgemäß wird eine Betätigungseinheit bereitgestellt, die räumlich getrennt von dem medizinischen Gerät sein kann. Dadurch lässt sich eine Betätigungseinheit an einem medizinischen Gerät oder ein Betätigungsbereich an dem medizinischen Gerät kleiner und übersichtlicher ausgestalten, als wenn auf dieser Betätigungseinheit bzw. dem Betätigungsbereich zusätzliche Betätigungselemente angebracht sein müssen, um die Versorgung mit Fluid zu verändern.

Erfindungsgemäß lässt sich eine Nachricht mit einer Betätigungsinformation über eine Datenverbindung drahtlos von dem Sender der oder einer Betätigungseinheit zu dem Empfänger der Versorgungseinheit übermitteln. Dieses Merkmal erspart die Notwendigkeit, die Betätigungseinheit durch ein Kabel oder eine sonstige Datenleitung mit der Versorgungseinheit zu verbinden. Die Länge dieser Datenleitung begrenzt den maximal möglichen Abstand zwischen der Betätigungseinheit und der Versorgungseinheit während einer Betätigung eines Betätigungselements der Betätigungseinheit. In vielen Fällen ist es leichter, die Entfernung zwischen der oder einer Betätigungseinheit und der Versorgungseinheit mithilfe einer drahtlosen Datenverbindung zwischen dem Sender und dem Empfänger zu überbrücken als mit einer Datenleitung.

Typischerweise umfasst die Versorgungseinheit ein Gehäuse. Gerade bei einem Einsatz des Versorgungssystems in einem Krankenhaus oder einer sonstigen medizinischen Umgebung muss dieses Gehäuse regelmäßig gereinigt und desinfiziert werden. Falls die Betätigungseinheit durch ein Kabel oder eine sonstige Datenleitung mit der Versorgungseinheit verbunden wäre, so müsste diese Datenleitung durch eine Aussparung in dem Gehäuse geführt werden. Diese Aussparung könnte ein Einfallstor für schädliche Partikel sein. Die Datenleitung müsste ebenfalls regelmäßig gereinigt und desinfiziert werden. Dies stellt zusätzliche Anforderungen an den Werkstoff, aus dem die Ummantelung der Datenleitung hergestellt ist. Insbesondere eine längere Datenleitung kann einen Aufroll-Mechanismus erfordern oder sich an einem anderen Gegenstand verhaken. Außerdem kann eine Datenleitung unter dem Einfluss einer mechanischen Belastung beschädigt werden.

Die Erfindung vermeidet die Nachteile einer solchen Datenleitung, weil eine Betätigungsinformation sich erfindungsgemäß durch eine drahtlose Datenverbindung übermitteln lässt. Insbesondere lässt ein erfindungsgemäßes Versorgungssystem sich leichter und mit einem geringeren Zeitaufwand reinigen und desinfizieren.

Möglich ist, dass eine Betätigung eines Betätigungselements der oder einer Betätigungseinheit den Schritt auslöst, dass zwischen dem Sender dieser Betätigungseinheit und dem Empfänger der Versorgungseinheit eine drahtlose Datenverbindung hergestellt wird und die Nachricht mit der Betätigungsinformation dann über diese hergestellte drahtlose Datenverbindung übermittelt wird. Danach kann die Datenverbindung wieder unterbrochen oder beendet werden. Möglich ist auch, dass die Datenverbindung während der Benutzung des medizinischen Geräts dauerhaft hergestellt ist.

In einer Ausgestaltung umfasst die Versorgungseinheit mehrere Fluid-Versorgungsanschlüsse. Diese können sich durch unterschiedliche bereitgestellte Fluide unterscheiden. Möglich ist auch, dass zwei Fluid-Versorgungsanschlüsse das gleiche Fluid mit unterschiedlichen Parametern bereitstellen, beispielsweise mit unterschiedlichen Flussraten und / oder unterschiedlichen Drücken relativ zum Umgebungsdruck und / oder unterschiedlichen Temperaturen. Optional umfasst die Versorgungseinheit mindestens einen weiteren Versorgungsanschluss für elektrische Energie oder für die Anbindung des medizinischen Geräts an ein Datennetz.

Erfindungsgemäß vermag der Sender einer Betätigungseinheit eine Nachricht mit einer Betätigungsinformation an den Empfänger der Versorgungseinheit zu übermitteln. In einer bevorzugten Ausgestaltung umfasst diese Betätigungsinformation eine Kennzeichnung des Fluid-Versorgungsanschlusses, auf den sich die Betätigung bezieht. Im Falle von mehreren Fluid-Versorgungsanschlüssen und auch im Falle von nur einem Fluid-Versorgungsanschluss umfasst die Betätigungsinformation bevorzugt zusätzlich eine Nachricht mit einer gewünschten Handlung an diesem Fluid-Versorgungsanschluss, insbesondere die Festlegung, ob der Volumenfluss vergrößert oder verkleinert werden soll. Ein Sonderfall ist die Betätigungsinformation, dass die Bereitstellung von Fluid durch diesen Fluid-Versorgungsanschluss begonnen werden, also von null auf einen Wert größer null erhöht werden soll, oder beendet werden, also der Volumenfluss auf null reduziert werden soll. Ein weiterer Sonderfall ist die Betätigungsinformation, dass die Bereitstellung von Fluid auf den größtmöglichen Wert erhöht werden soll. Möglich ist auch, dass ein Benutzer ein Betätigungselement so lange und/oder so oft betätigt, bis ein Parameter des oder eines Fluid-Versorgungsanschlusses einen gewünschten Wert annimmt. Bevorzugt wird der aktuell erreichte Wert in einer von einem Menschen wahrnehmbaren Form ausgegeben.

Ein medizinisches Gerät lässt sich bevorzugt lösbar mit dem oder mindestens einem Fluid-Versorgungsanschluss der Versorgungseinheit verbinden. Dadurch lassen sich verschiedene medizinische Geräte, auch unterschiedliche medizinische Geräte, nacheinander mit derselben Versorgungseinheit verbinden und mit Fluid versorgen.

Die Versorgungseinheit ist bevorzugt ortsfest, also stationär ausgestaltet. Sie lässt sich insbesondere in einem medizinisch genutzten Bereich, z.B. in einem Patientenzimmer, einem Operationssaal und / oder einer Intensivstation eines Krankenhauses, in einem Behandlungszimmer einer Arztpraxis oder auch in einem Rettungsfahrzeug, verwenden. Ein fahrbares oder auf andere Weise transportables Gerät lässt sich zeitweise mit der Versorgungseinheit verbinden und kann dann mit dem oder einem Fluid versorgt werden.

In einer Ausgestaltung umfasst die Versorgungseinheit einen Anschlusskörper, der an einem freien Ende eines Montagearms befestigt ist, insbesondere schwenkbar an einem Schwenkarm. Der oder jeder Fluid-Versorgungsanschluss ist in den Anschlusskörper eingelassen. Ein anderes freies Ende dieses Montagearms ist an einer Decke oder einer Wand eines medizinischen Bereichs befestigt. Der Montagearm ist so ausgestaltet, dass der Anschlusskörper der Versorgungseinheit sich relativ zu der Decke oder der Wand, an welcher der Montagearm befestigt ist, bewegen lässt. Dadurch lässt der Anschlusskörper sich in eine gewünschte Position verbringen, soweit dies der Montagearm erlaubt.

In einer Ausgestaltung umfasst die Versorgungseinheit eine erste Aufnahmeeinheit und optional zusätzlich mindestens eine zweite Aufnahmeeinheit, wobei die oder jede zweite Aufnahmeeinheit mit einem Abstand zur ersten Aufnahmeeinheit positioniert ist. Die oder jede Aufnahmeeinheit vermag jeweils eine Betätigungseinheit aufzunehmen. Bevorzugt lässt sich die oder eine Betätigungseinheit in die Aufnahmeeinheit verbringen und wieder aus der Aufnahmeeinheit entnehmen oder auf andere Weise von der Aufnahmeeinheit trennen. Ein Benutzer kann die oder eine Betätigungseinheit aus der oder einer Aufnahmeeinheit entnehmen, sich zu einem gewünschten Ort begeben, beispielsweise in die Nähe eines Patienten oder einer Anzeigeeinheit des medizinischen Geräts, von dort mindestens einmal mindestens einen Fluid-Versorgungsanschluss betätigen und anschließend die Betätigungseinheit wieder in die oder eine Aufnahmeeinheit verbringen. Möglich, aber dank der Ausgestaltung mit der Aufnahmeeinheit nicht erforderlich ist, dass ein Benutzer dauerhaft eine Betätigungseinheit mit sich führt.

Möglich ist, dass das Versorgungssystem genauso viele oder mehr oder weniger Betätigungseinheiten umfasst als die Versorgungseinheit Aufnahmeeinheiten bereitstellt.

Unterschiedliche Ausgestaltungen sind möglich, wie eine Aufnahmeeinheit eine Betätigungseinheit hält. Beispielsweise umfasst die Aufnahmeeinheit eine Tasche, in welche sich die oder eine Betätigungseinheit ablegen lässt. Oder sie umfasst mindestens einen Vorsprung, und die Betätigungseinheit umfasst eine entsprechende Aussparung, oder umgekehrt die Betätigungseinheit einen Vorsprung und die Aufnahmeeinheit eine Aussparung. Der Vorsprung umfasst beispielsweise einen Haken, die Aussparung eine Öse. Möglich ist auch, dass die Aufnahmeeinheit die Betätigungseinheit mithilfe eine Rastverbindung, einer Klemmverbindung, einer Klettverbindung oder eines Permanentmagneten oder Elektromagneten hält.

In einer Ausgestaltung umfasst die Versorgungseinheit ein Gehäuse mit mindestens zwei Außenwand-Abschnitten. Die beiden Außenwand-abschnitte können sich in zwei Ebenen erstrecken, welche senkrecht oder schräg aufeinander stehen und bevorzugt beide vertikal angeordnet sind. Bevorzugt ist an jedem dieser beiden Außenwand-Abschnitte jeweils mindestens eine Aufnahmeeinheit angeordnet. Dank dieser Ausgestaltung lässt sich aus mindestens zwei unterschiedlichen Richtungen jeweils mindestens eine Betätigungseinheit greifen, ohne um die Versorgungseinheit herumgehen zu müssen und ohne die Versorgungseinheit drehen zu müssen. Möglich ist, dass an diesen beiden Außenwand-Abschnitten zusätzlich jeweils mindestens ein Fluid-Versorgungsanschluss angebracht ist. In einer Fortbildung dieser Ausgestaltung hat das Gehäuse die Form einer quaderförmigen Säule, optional einer Säule mit einem quadratischen Grundriss. Diese Säule stellt vier Außenwand-Abschnitte bereit. Bevorzugt ist an mindestens drei dieser vier Außenwand-Abschnitte jeweils mindestens eine Aufnahmeeinheit angeordnet, besonders bevorzugt an allen vier Außenwand-Abschnitten.

In einer Ausgestaltung umfasst das Versorgungssystem genau eine Betätigungseinheit. Mit dieser Betätigungseinheit lässt sich jeder Fluid-Versorgungsanschluss der Versorgungseinheit betätigen - oder wenigstens jeder Fluid-Versorgungsanschluss, der sich aus einer Entfernung ansteuern und betätigen lässt.

In einer anderen Ausgestaltung umfasst das Versorgungssystem mehrere Betätigungseinheiten. Jede Betätigungseinheit umfasst jeweils einen Sender. Zwischen dem jeweiligen Sender jeder Betätigungseinheit und dem Empfänger der Versorgungseinheit lässt sich wenigstens zeitweise eine Datenverbindung herstellen, und der Sender vermag über diese Datenverbindung eine Nachricht mit einer Betätigungsinformation drahtlos an den Empfänger zu übermitteln. Dieselbe Versorgungseinheit lässt sich dadurch von verschiedenen Betätigungseinheiten und dadurch auch von unterschiedlichen Personen betätigen, auch gleichzeitig oder zeitlich überlappend.

In einer Ausgestaltung ist jedem Fluid-Versorgungsanschluss, der sich über eine Entfernung hinweg betätigen lässt, jeweils eine eigene Betätigungseinheit zugeordnet. Umgekehrt lässt sich bei dieser Ausgestaltung mit jeder Betätigungseinheit jeweils genau ein Fluid-Versorgungsanschluss betätigen.

In einer anderen Ausgestaltung wird hingegen ermöglicht, dass es weniger Betätigungseinheiten als Fluid-Versorgungsanschlüsse gibt. Weiterhin wird ermöglicht, dass sich derselbe Fluid-Versorgungsanschluss durch unterschiedliche, auch unterschiedlich positionierte Betätigungseinheiten betätigen lässt.

In einer bevorzugten Realisierung dieser anderen Ausgestaltung lässt sich zu jedem Zeitpunkt nur eine Datenverbindung zwischen einem Sender einer Betätigungseinheit und dem Empfänger der Versorgungseinheit herstellen. Mit anderen Worten: Solange eine Datenverbindung zwischen dem Sender einer Betätigungseinheit und dem Empfänger hergestellt ist, ist es nicht möglich, eine Datenverbindung zwischen dem Sender einer anderen Betätigungseinheit und demselben Empfänger herzustellen. Vielmehr sperrt und verhindert das Versorgungssystem die Herstellung dieser weiteren Datenverbindung. Dank dieser Ausgestaltung ist sichergestellt, dass dieselbe Versorgungseinheit nicht gleichzeitig oder zeitlich überlappend von verschiedenen Betätigungseinheiten betätigt und verändert wird. Verhindert wird damit insbesondere, dass gleichzeitig mehrere Personen dieselbe Versorgungseinheit und insbesondere denselben Fluid-Versorgungsanschluss betätigen.

Bevorzugt speichert die Versorgungseinheit in einem Datenspeicher eine eindeutige Kennung derjenigen Betätigungseinheit ab, deren Sender aktuell durch eine drahtlose Datenverbindung mit dem Empfänger der Versorgungseinheit verbunden ist, oder die Information, dass aktuell keine drahtlose Datenverbindung hergestellt ist. Die eindeutige Kennung unterscheidet diese Betätigungseinheit von jeder anderen Betätigungseinheit des Versorgungssystems.

In einer bevorzugten Weiterentwicklung dieser Ausgestaltung umfasst jede Betätigungseinheit jeweils eine erste Pairing-Einheit. Die Versorgungseinheit umfasst eine zweite Pairing-Einheit. Beim Schritt, eine Datenverbindung zwischen dem Sender einer Betätigungseinheit und dem Empfänger der Versorgungseinheit herzustellen, versuchen die beiden Pairing-Einheiten, ein Pairing durchzuführen. Gelingt dieses Pairing, so ist die Datenverbindung hergestellt. Solange diese Datenverbindung hergestellt ist, ist der Vorgang gesperrt, dass die erste Pairing-Einheit einer anderen Betätigungseinheit und die zweite Pairing-Einheit erfolgreich ein Pairing durchführen und dadurch eine weitere Datenverbindung realisieren. Außerdem lässt sich dadurch der unerwünschte Vorgang verhindern, dass ein Benutzer mit einer anderen Betätigungseinheit oder einem anderen Gerät ungewollt oder auch unbefugt die Versorgungseinheit betätigt.

Erfindungsgemäß vermag das Versorgungssystem wenigstens zeitweise eine drahtlose Datenverbindung zwischen dem Sender der oder einer Betätigungseinheit und dem Empfänger der Versorgungseinheit herzustellen. Über diese Datenverbindung lässt sich eine Nachricht mit einer Betätigungsinformation von dem Sender zu dem Empfänger übermitteln. In einer Ausgestaltung vermag umgekehrt der Empfänger eine Nachricht an den Sender zu übermitteln. In einer Ausgestaltung übermittelt der Empfänger der Versorgungseinheit eine Nachricht mit einer Bestätigungsinformation an den Sender, sobald die in der Betätigungsinformation spezifizierte Betätigung des oder eines Fluid-Versorgungsanschlusses durchgeführt wurde. Alternativ übermittelt der Empfänger eine Fehlermeldung an den Sender, falls diese Betätigung nicht durchgeführt werden konnte. Bevorzugt umfasst die Betätigungseinheit eine Ausgabeeinheit, um eine empfangene Nachricht in einer von einem Menschen wahrnehmbaren Form auszugeben. Diese Ausgestaltung reduziert insbesondere das Risiko, dass ein Benutzer meint, einen Fluid-Versorgungsanschluss betätigt zu haben, dieser Fluid-Versorgungsanschluss in Wirklichkeit aber nicht betätigt wurde.

In einer Ausgestaltung übermittelt der Sender einer Betätigungseinheit eine Nachricht mit einer Betätigungsinformation in einer verschlüsselten Form. Bevorzugt übermittelt der Empfänger der Versorgungseinheit einen rechnerauswertbaren Schlüssel an den Sender. Der Sender verwendet den empfangenen Schlüssel, um eine Nachricht mit einer Betätigungsinformation zu verschlüsseln. Diese Ausgestaltung reduziert weiter das Risiko, dass ein Fluid-Versorgungsanschluss von einer nicht autorisierten Person betätigt wird.

In einer Fortbildung dieser Ausgestaltung umfasst das Versorgungssystem mindestens zwei Versorgungseinheiten mit jeweils einem Empfänger. Jeder Empfänger vermag eine Nachricht an den Sender der oder einer Betätigungseinheit verschlüsselt zu übermitteln. Die beiden Versorgungseinheiten besitzen unterschiedliche Schlüssel. Die Betätigungseinheit verwendet einen empfangenen Schlüssel einer Versorgungseinheit, um eine Nachricht mit einer Betätigungsinformation an diese Versorgungseinheit verschlüsselt zu übermitteln. In einer Ausgestaltung lassen sich beide Versorgungseinheiten mit derselben Betätigungseinheit betätigen
Die Ausgestaltung mit den unterschiedlichen Schlüsseln reduziert weiter die Gefahr, dass ein Fluid-Versorgungsanschluss ungewollt oder unbefugt betätigt wird.

In einer Ausgestaltung wird ein Aktivierungs-Kriterium vorgegeben, welches sich auf die Position, Orientierung, Bewegung und / oder Beschleunigung einer Betätigungseinheit relativ zu der Versorgungseinheit und / oder auf den Abstand zwischen der Betätigungseinheit und der Versorgungseinheit bezieht. Eine Datenverbindung zwischen dem Sender der oder einer Betätigungseinheit und dem Empfänger der Versorgungseinheit lässt sich nur dann herstellen, wenn dieses Aktivierungs-Kriterium erfüllt ist. Ansonsten ist die Herstellung der Datenverbindung nicht möglich.

Das Versorgungssystem umfasst gemäß einer bevorzugten Realisierung dieser Ausgestaltung einen Positionssensor, der die Position, Orientierung, Bewegung und / oder Beschleunigung einer Betätigungseinheit relativ zu der Versorgungseinheit misst. Dieser Positionssensor kann ein Bestandteil der Betätigungseinheit oder ein Bestandteil der Versorgungseinheit sein. Ein Steuergerät der Betätigungseinheit und / oder ein Steuergerät der Versorgungseinheit empfängt Signale des Positionssensors und prüft abhängig von diesen Signalen, ob das vorgegebene Aktivierungs-Kriterium erfüllt ist oder nicht. Diese Ausgestaltung reduziert weiter die Gefahr, dass eine unerwünschte oder unbefugte Betätigung eines Fluid-Versorgungsanschlusses ausgelöst wird.

Falls das Aktivierungs-Kriterium erfüllt ist, so lässt sich eine drahtlose Datenverbindung herstellen. Bevorzugt misst der Positionssensor auch bei hergestellter Datenverbindung mindestens einmal die relative Position, Orientierung, Bewegung und / oder Beschleunigung, und das Steuergerät prüft abhängig von Signalen des Positionssensors, ob das Aktivierungs-Kriterium noch erfüllt ist. Bevorzugt unterbricht das Steuergerät die hergestellte Datenverbindung, wenn das Aktivierungs-Kriterium nicht mehr erfüllt ist. Diese Ausgestaltung reduziert weiter die Gefahr einer unerwünschten oder ungewollten oder unbefugte Betätigung.

Eine Ausgestaltung der Erfindung ermöglicht es in vielen Fällen, sicherzustellen, dass ein Fluid-Versorgungsanschluss nur dann betätigt wird, wenn der Abstand zwischen der Betätigungseinheit und der Versorgungseinheit nicht größer als ein vorgegebener Maximal-Abstand ist und / oder wenn sich die Betätigungseinheit und die Versorgungseinheit in demselben Raum befinden. Diese Ausgestaltung reduziert die Gefahr, dass ein Benutzer ungewollt oder auf ungewollte Weise einen Fluid-Versorgungsanschluss betätigt, was auch dann passieren kann, wenn dieser Benutzer als ein autorisierter Benutzer verifiziert ist.

Bei dieser Ausgestaltung wird die Reichweite des Senders auf eine bestimmte Maximal-Sendeleistung beschränkt, bevorzugt auf eine Sendeleistung in einem Bereich von -6 dBm bis 3 dBm, insbesondere auf ca. 0 dBm. Die maximale Sendeleistung lässt sich beispielsweise so ausgestalten, dass die vom Sender ausgestrahlten elektromagnetischen Wellen nicht eine Wand durchdringen können und / oder die Entfernung, welche die ausgestrahlten elektromagnetischen Wellen überbrücken können, höchstens gleich dem Maximalabstand ist.

Erfindungsgemäß lässt sich wenigstens zeitweise eine drahtlose Datenverbindung zwischen dem Sender einer Betätigungseinheit und dem Empfänger der Versorgungseinheit herstellen. In einer Ausgestaltung wird diese Datenverbindung gemäß mindestens einem der folgenden Übertragungsverfahren hergestellt:
- Bluetooth, insbesondere Bluetooth Advertising,
- WLAN
- Certified Wireless USB
- ZigBee
- Z-Wave
- DECT
- WirelessHART
- HiperLAN
- HomeRF
- LoRaWAN.

Möglich ist, dass zunächst ein erstes Übertragungsverfahren verwendet wird. Scheitert der Versuch, eine drahtlose Datenübermittlung über dieses erste Verfahren herzustellen, so wird ein zweites Übertragungsverfahren verwendet. Möglich ist auch, dass es von einer gemessenen Umgebungsbedingung abhängt, welches Übertragungsverfahren angewendet wird. Die Umgebungsbedingung kann beispielsweise davon abhängen, welche elektronischen Geräte sich in der Nähe der Betätigungseinheit befinden, die zur Betätigung verwendet wird.

Bevorzugt umfasst die oder jede Betätigungseinheit jeweils eine eigene Spannungsversorgungseinheit. Bevorzugt umfasst die Versorgungseinheit mindestens eine Dockingstation, in die sich eine Betätigungseinheit einsetzen lässt. Nach dem Einsetzen wird die Spannungsversorgungseinheit der eingesetzten Betätigungseinheit aufgeladen. Diese Ausgestaltung erspart die Notwendigkeit, die Spannungsversorgungseinheit zum Aufladen aus der Betätigungseinheit herausnehmen zu müssen. Möglich ist, in derselben Dockingstation nacheinander unterschiedliche Betätigungseinheiten aufzuladen.

Bevorzugt lässt sich die oder jede Betätigungseinheit wahlweise in einem aktivierten Modus oder in einem Standby-Modus betreiben. Im aktivierten Modus vermag die Betätigungseinheit eine Betätigung eines Betätigungselements zu erfassen und eine Nachricht mit einer Betätigungsinformation an den Empfänger zu übermitteln. Im Standby-Modus verbraucht die Betätigungseinheit weniger elektrische Energie pro Zeiteinheit als im aktivierten Modus. Diese Ausgestaltung verlängert die Einsatzdauer der Betätigungseinheit verglichen mit einer Ausgestaltung, bei der die Betätigungseinheit dauerhaft in einem aktivierten Modus ist.

Bevorzugt geht die Betätigungseinheit automatisch in den Standby-Modus über, wenn im Verlaufe einer Zeitspanne von einer vorgegebenen Mindestdauer kein Betätigungselement betätigt worden ist. Bevorzugt geht die Betätigungseinheit vom Standby-Modus automatisch wieder in den aktivierten Modus über, d.h. wird "aufgeweckt", wenn mindestens eines der folgenden Ereignisse detektiert ist:
- Das oder ein Betätigungselement der Betätigungseinheit wurde betätigt.
- Ein Bewegungssensor der Betätigungseinheit detektiert eine Bewegung der Betätigungseinheit.
- Ein Näherungssensor detektiert, dass ein Lebewesen und / oder ein Objekt sich der Betätigungseinheit nähert. Der Näherungssensor kann beispielsweise die Temperatur messen und dadurch erkennen, dass die Hand eines Benutzers nach der Betätigungseinheit greift.
- Der Sender der Betätigungseinheit vermag zusätzlich Nachrichten zu empfangen, und zwar auch im Standby-Modus, und eine Aktivierungs-Nachricht wurde an diesem Sender übermittelt.

Möglich ist, die Betätigungseinheit so auszugestalten, dass sie bei einer gewünschten Betätigung ausreichend rasch in den aktivierten Modus übergeht.

In einer Ausgestaltung umfasst die Betätigungseinheit eine Verifizierungseinheit. Mithilfe dieser Verifizierungseinheit kann ein Benutzer automatisch als autorisierter Benutzer der Betätigungseinheit verifiziert werden - oder festgestellt werden kann, dass ein Benutzer nicht autorisiert ist. Die Verifizierungseinheit erfasst beispielsweise eine eingegebene oder gesprochene alphanumerische Zeichenfolge und vergleicht diese mit mindestens einer abgespeicherten alphanumerischen Zeichenfolge, die einem autorisierten Benutzer zugeordnet ist. Oder die Verifizierungseinheit erfasst ein biometrisches Merkmal, beispielsweise einen Fingerabdruck oder ein Merkmal der Iris, und vergleicht dieses erfasste biometrisches Merkmal mit einem abgespeicherten biometrischen Merkmal, welches einem autorisierten Benutzer zugeordnet ist. Nur dann, wenn ein Benutzer als autorisierter Benutzer verifiziert worden ist, kann dieser Benutzer die Betätigungseinheit benutzen, um einen Fluid-Versorgungsanschluss zu betätigen.

In einer Ausgestaltung kann ein als autorisiert erkannter Benutzer der Betätigungseinheit jeden Fluid-Versorgungsanschluss der Versorgungseinheit betätigen. In einer anderen Ausgestaltung ist jedem registrierten autorisierten Benutzer eine Festlegung zugeordnet, welche Fluid-Versorgungsanschlüsse dieser Benutzer betätigen darf und welche nicht. Beispielsweise dürfen bestimmte Benutzer jeden Fluid-Versorgungsanschluss betätigen, andere Benutzer nur jeweils einen Fluid-Versorgungsanschluss oder einige Fluid-Versorgungsanschlüsse. Durch diese Ausgestaltung lässt sich sicherstellen, dass nur ein ausreichend qualifizierter und berechtigter Benutzer einen Fluid-Versorgungsanschluss betätigt. Andererseits werden qualifizierte Benutzer entlastet, weil bestimmte Fluid-Versorgungsanschlüsse auch von anderen Benutzern betätigt werden können.

Erfindungsgemäß umfasst die oder jede Betätigungseinheit jeweils mindestens ein Betätigungselement. Das oder mindestens ein Betätigungselement umfasst bevorzugt einen physischen Knopf und / oder einen Schalter und / oder eine Schaltfläche. Dieses Betätigungselement steht über ein Gehäuse der Betätigungseinheit über oder ragt aus dem Gehäuse hervor. Ein solches Betätigungselement lässt sich in vielen Fällen sicherer als ein auf andere Weise ausgestaltetes Betätigungselement betätigen, insbesondere wenn ein Benutzer einen Handschuh trägt und / oder wenn der Benutzer das Betätigungselement betätigt, ohne auf die Betätigungseinheit zu schauen, sondern beispielsweise auf einen Patienten oder auf die Versorgungseinheit oder auf eine Anzeigeeinheit, welche Vitalparameter des Patienten anzeigt.

Erfindungsgemäß umfasst die oder jede Betätigungseinheit jeweils mindestens ein Betätigungselement. In einer Ausgestaltung umfasst die oder mindestens eine Betätigungseinheit mehrere Betätigungselemente. Bevorzugt lässt sich jedes Betätigungselement unabhängig von dem oder jedem anderen Betätigungselement betätigen. Mehrere Betätigungselemente einer Betätigungseinheit können sich auf unterschiedliche Fluid-Versorgungsanschlüsse beziehen und / oder auf verschiedene Parameter desselben Fluid-Versorgungsanschlusses.

Bevorzugt umfassen die Versorgungseinheit und die oder jede Betätigungseinheit jeweils ein Gehäuse. Jedes Gehäuse ist bevorzugt so ausgestaltet, dass das Gehäuse die für ein Gehäuse relevanten Anforderungen an ein medizinisches Gerät erfüllt, insbesondere an Forderungen hinsichtlich der Reinigung und Desinfizierung eines Geräts.

Im Folgenden wird die Erfindung anhand eines Ausführungsbeispiels beschrieben. Hierbei zeigen
- Figur 1: eine erste Ausführungsform des Versorgungssystems, wobei die Betätigungseinheiten fortgelassen sind;
- Figur 2: eine zweite Ausführungsform des Versorgungssystems, wobei die Betätigungseinheiten gezeigt sind.

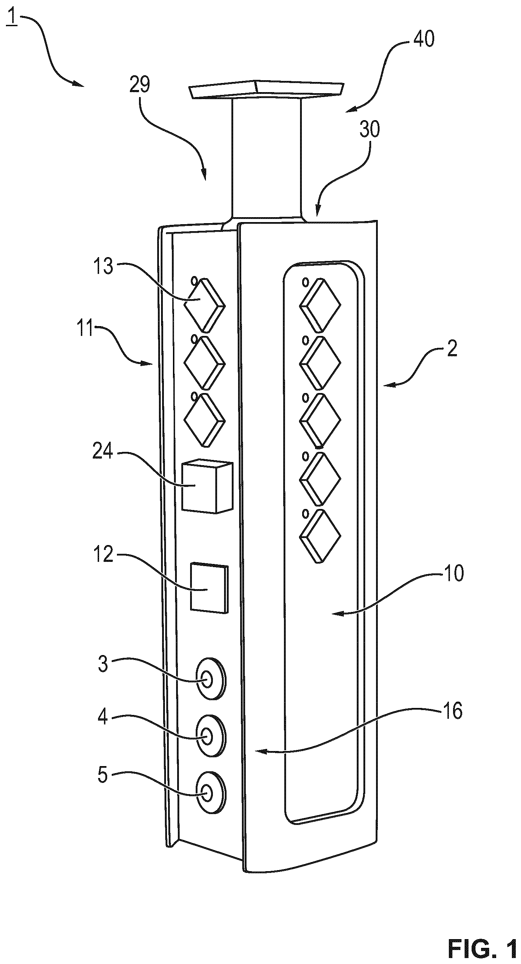

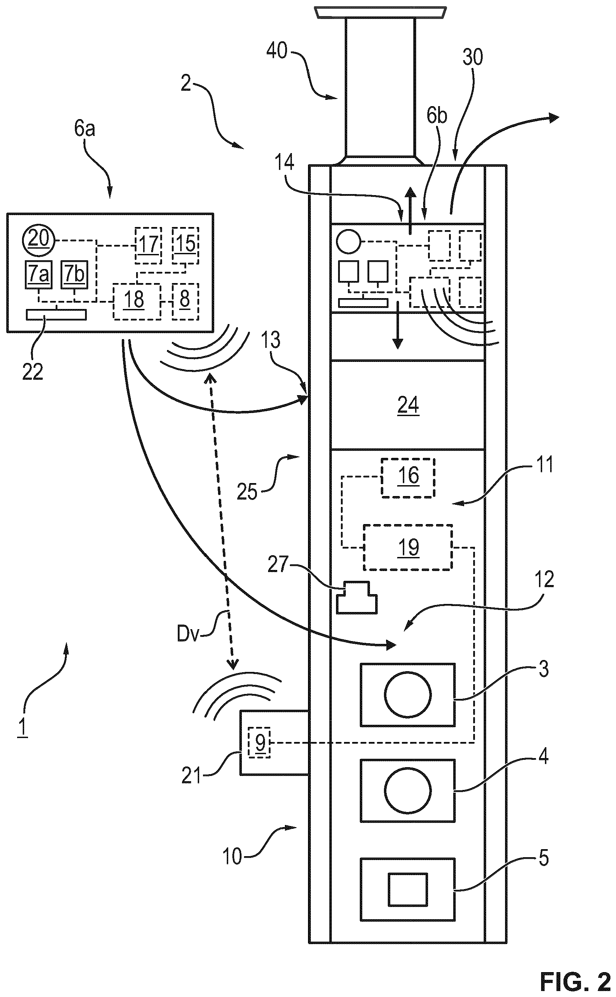

Figur 1 und Figur 2 zeigen schematisch zwei leicht differierende Ausführungsformen eines Versorgungssystem 1, welches ein nicht gezeigtes medizinisches Gerät mit unterschiedlichen Versorgungsmedien, u.a. mit verschiedenen Fluiden, also Gase oder Flüssigkeiten, zu versorgen vermag. Das medizinische Gerät ist beispielsweise ein Beatmungsgerät oder ein Anästhesiegerät oder ein Patienten-Monitor. Eine ortsfeste Versorgungseinheit 2 ist beispielsweise an einer Wand oder Decke eines Krankenhaussaals befestigt und umfasst einen Anschlusskörper 25 mit mehreren Außenwand-Abschnitten 11, 12.

Bevorzugt hat dieser Anschlusskörper 25 die Form einer Säule mit einem quadratischen oder anderweitig rechteckigen Grundriss. Der Anschlusskörper 25 hängt an einem Montagearm 40, der in den Figuren nur teilweise dargestellt ist. Eine nach oben zeigende Stirnseite 30 des Anschlusskörpers 25 ist über einen Montagebereich 29 mit dem Montagearm 40 verbunden.

In diesen Anschlusskörper 25 sind mehrere Fluid-Versorgungsanschlüsse 3, 4, 5 integriert. Jeder Fluid-Versorgungsanschluss 3, 4, 5 vermag ein Versorgungsmedium in Form eines Fluids bereitzustellen und lässt sich lösbar mit jeweils einer korrespondierenden Versorgungsleitung des medizinischen Geräts verbinden. Im Ausführungsbeispiel vermag der Versorgungsanschluss 3 Sauerstoff bereitzustellen, der Versorgungsanschluss 4 Atemluft mit einer niedrigeren Flussrate und der Versorgungsanschluss 5 Atemluft mit einer höheren Flussrate, insgesamt also zwei unterschiedliche Fluide. Ein weiterer Versorgungsanschluss 27 ist ein Netzwerkanschluss, beispielsweise eine RJ45-Steckdose für eine Ethernet-Verbindung. Indem ein RJ45-Stecker in diese Steckdose gesteckt wird, lässt sich das medizinische Gerät an ein drahtgebundenes Datennetz des Krankenhauses anschließen. Optional umfasst der Anschlusskörper 25 mindestens einen nicht gezeigten elektrischen Versorgungsanschluss in Form einer Steckdose, um elektrische Energie bereitzustellen, und optional einen Griff, um die Position des Anschlusskörpers 25 relativ zu dem Montagearm 40 zu verändern.

Zum Versorgungssystem 1 gehören weiterhin zwei Betätigungseinheiten 6a und 6b, die nur in Figur 2 dargestellt sind und im Ausführungsbeispiel als tragbare Geräte nach Art einer Fernbedienung ausgestaltet sind und gleichartig aufgebaut sein können oder sich voneinander unterscheiden können. Optional kann das Versorgungssystem 1 mindestens eine weitere Betätigungseinheit umfassen. Jede Betätigungseinheit 6a, 6b umfasst eine eigene Spannungsversorgungseinheit, bevorzugt einen Akku oder einen Akkupack. An dem Anschlusskörper 25 sind im Ausführungsbeispiel drei Aufnahmeeinheiten 12, 13, 14 und optional mindestens eine weitere, nicht gezeigte Aufnahmeeinheit angeordnet. An einem zum Betrachter von Figur 1 zeigenden vorderen Außenwand-Abschnitt 11 sind die beiden Aufnahmeeinheiten 12 und 14 angeordnet, an einem seitlichen Außenwand-Abschnitt 10 die Aufnahmeeinheit 13. Die Aufnahmeeinheiten 12, 13, 14 können je eine Tasche umfassen, in welche sich die Betätigungseinheit 6a, 6b hineinstecken lässt. In einer alternativen Ausgestaltung hält die Aufnahmeeinheit eine Betätigungseinheit 6a, 6b mithilfe eines Permanentmagneten oder Elektromagneten. Eine derartige Aufnahmeeinheit lässt sich leichter als anders ausgestaltete Aufnahmeeinheiten desinfizieren und reinigen.

Jede Aufnahmeeinheit 12, 13, 14 vermag jeweils eine Betätigungseinheit 6a, 6b aufzunehmen. Eine Betätigungseinheit 6a, 6b lässt sich wahlweise in eine Aufnahmeeinheit 12, 13, 14 einstecken oder auf andere Weise einsetzen und wieder aus dieser Aufnahmeeinheit 12, 13, 14 entnehmen. Bevorzugt lässt sich jede Betätigungseinheit 6a, 6b wahlweise in jede der Aufnahmeeinheiten 12, 13, 14 einsetzen. Eine Aufnahmeeinheit 12, 13, 14 verhindert, dass eine eingesetzte Betätigungseinheit 6a, 6b von allein herausfällt.

Der Anschlusskörper 25 umfasst weiterhin eine Dockingstation 24 an dem vorderen Außenwand-Abschnitt 11 und optional eine weitere, nicht gezeigte Dockingstation. Die Dockingstation 24 weist eine Kontaktfläche auf. Jede Betätigungseinheit 6a, 6b weist eine korrespondierende Kontaktfläche auf. Wenn die Betätigungseinheit 6a, 6b in die Dockingstation 24 oder in eine andere Dockingstation eingesetzt ist, wird die Spannungsversorgungseinheit der eingesetzten Betätigungseinheit 6a, 6b über die beiden Kontaktflächen aufgeladen. Alternativ vermag die Dockingstation 24 die Spannungsversorgungseinheit auch induktiv und damit berührungslos aufzuladen.

Die Betätigungseinheit 6a umfasst zwei Betätigungselemente 7a, 7b und optional weitere, nicht gezeigte Betätigungselemente, welche ein Benutzer betätigen kann, um von außen einen Fluid-Versorgungsanschluss 3, 4, 5 anzusteuern und dadurch den Volumenfluss des Fluids durch den Fluid-Versorgungsanschluss 3, 4, 5 zu verändern. Insbesondere kann der Benutzer durch diese Betätigung den Volumenfluss einschalten oder ausschalten sowie den Volumenfluss vergrößern oder verkleinern. In einer Ausgestaltung ist jedes Betätigungselement 7a, 7b jeweils einem Fluid-Versorgungsanschluss 3, 4, 5 zugeordnet. In einer anderen Ausgestaltung wählt der Benutzer mit einem Betätigungselement 7a zuerst einen Fluid-Versorgungsanschluss 3, 4, 5 aus und verändert dann mit einem anderen Betätigungselement 7b den Volumenfluss. Im Ausführungsbeispiel ist die Betätigungseinheit 6b genauso wie die Betätigungseinheit 6a aufgebaut
Die Betätigungseinheit 6a umfasst außerdem ein datenverarbeitendes Steuergerät (controller) 18 und einen Sender 8. Die oder jede andere Betätigungseinheit 6b umfasst ebenfalls jeweils ein Steuergerät und einen Sender. Das Steuergerät 18 vermag eine Betätigung eines Betätigungselements 7a, 7b zu erfassen und eine Nachricht mit einer der Betätigung entsprechenden Betätigungsinformation zu generieren. Diese Betätigungsinformation umfasst die Festlegung eines Fluid-Versorgungsanschlusses 3, 4, 5 sowie eine verlangte Betätigung dieses Fluid-Versorgungsanschlusses 3, 4, 5. Die Versorgungseinheit 2 umfasst ein Steuergerät (controller) 19 und einen Empfänger 9. Der Empfänger 9 ist an einem Vorsprung 21 angeordnet. Zwischen dem Sender 8 einer Betätigungseinheit 6a, 6b und dem Empfänger 9 der Versorgungseinheit 2 lässt sich wenigstens zeitweise eine drahtlose Datenverbindung Dv, insbesondere per Funkwellen, herstellen. Über diese Datenverbindung Dv vermag der Sender 8 eine Nachricht mit einer Betätigungsinformation an den Empfänger 9 zu übermitteln. Das Steuergerät 19 der Versorgungseinheit 2 empfängt diese Nachricht und wertet sie aus und steuert den spezifizierten Fluid-Versorgungsanschluss 3, 4, 5 so wie in der empfangenen Nachricht spezifiziert an.

Im Ausführungsbeispiel wird sichergestellt, dass zu jedem Zeitpunkt höchstens eine Betätigungseinheit 6a, 6b eine Nachricht an den Empfänger 9 der Versorgungseinheit 2 schicken kann. Selbstverständlich können nacheinander mehrere Betätigungseinheiten 6a, 6b jeweils eine Nachricht an den Empfänger 9 übermitteln. Die Betätigungseinheit 6a umfasst eine Pairing-Einheit 15, die Versorgungseinheit 2 eine Pairing-Einheit 16. Bevor eine Nachricht von der Betätigungseinheit 6a an die Versorgungseinheit 2 übermittelt werden kann, führen die beiden Pairing-Einheiten 15 und 16 ein Pairing durch und stellen dadurch eine exklusive drahtlose Datenverbindung Dv zwischen der Betätigungseinheit 6a und der Versorgungseinheit 2 her. "Exklusiv" bedeutet, dass keine andere Betätigungseinheit eine Nachricht an die Versorgungseinheit 2 übermitteln kann, so lange diese exklusive Datenverbindung Dv hergestellt ist. Diese Datenverbindung Dv wird unterbrochen oder beendet, wenn ein vorgegebenes Abbruch-Kriterium erfüllt ist, beispielsweise wenn in einer Zeitspanne von einer vorgegebenen Mindestdauer keine Daten auf dieser Datenverbindung Dv mehr übermittelt worden sind. Das Entsprechende gilt für die Betätigungseinheit 6b.

Verhindert werden soll, dass eine Betätigungseinheit ungewollt einen Fluid-Versorgungsanschluss 3, 4, 5 der Versorgungseinheit 2 betätigt, beispielsweise weil der Benutzer dieser Betätigungseinheit in Wirklichkeit einen anderen Fluid-Versorgungsanschluss derselben Versorgungseinheit, eine andere Versorgungseinheit oder ein anderes Gerät betätigen will. Weiterhin soll eine missbräuchliche Betätigung verhindert werden. Im Ausführungsbeispiel werden mehrere Mechanismen angewendet, um dies zu verhindern. Diese Mechanismen werden am Beispiel der Betätigungseinheit 6a erläutert. Bevorzugt besitzen die Betätigungseinheit 6b und eine optionale dritte Betätigungseinheit ebenfalls mindestens einige oder alle dieser Sicherheitsmechanismen.

Die Betätigungseinheit 6a umfasst eine Verifizierungseinheit 22. Diese überprüft, ob ein Benutzer der Betätigungseinheit 6a ein autorisierter Benutzer ist, also ein Benutzer, der berechtigt ist, mithilfe der Betätigungseinheit 6a Betätigungsbefehle an die Versorgungseinheit 2 zu übermitteln. Die Verifizierungseinheit 22 erfasst beispielsweise ein Passierwort (password) oder eine PIN von dem Benutzer oder führt eine biometrische Prüfung durch. Die Betätigungselemente 7a, 7b sind gesperrt, bis ein Benutzer als ein autorisierter Benutzer erkannt worden ist.

Die Berechtigung des Benutzers kann sich auf alle Versorgungseinheiten und alle Fluid Versorgungsanschlüsse dieser Versorgungseinheiten erstrecken oder auf bestimmte Versorgungseinheiten und / oder auf bestimmte Fluid-Versorgungsanschlüsse 3, 4, 5 und / oder auf bestimmte Arten der Betätigung eingeschränkt sein. Optional sperrt die Betätigungseinheit 6a ein Betätigungselement 7a, 7b, wenn der aktuelle Benutzer nicht dafür autorisiert ist, mithilfe dieses Betätigungselements 7a, 7b eine Betätigung auszulösen.

Der Sender 8 der Betätigungseinheit 6a ist im Ausführungsbeispiel auf eine Sendeleistung in einem Bereich von -6 dBm bis 3 dBm (decibel milliwatts), insbesondere auf ca. 0dBm, beschränkt. Dadurch wird erreicht, dass die Betätigungseinheit 6a nur dann die Versorgungseinheit 2 ansteuern kann, wenn zwischen der Betätigungseinheit 6a und der Versorgungseinheit 2 ein Abstand nicht größer als ein vorgegebener maximaler Abstand eingehalten wird. Außerdem ist es dank dieser Ausgestaltung in der Regel nicht möglich, dass eine Nachricht mit einer Betätigungsinformation eine Wand durchdringt. Dadurch wird verhindert, dass die Betätigungseinheit 6a eine Versorgungseinheit betätigt, die sich aktuell in einem anderen Raum befindet. Eine solche Aktivierung aus einem Raum heraus in einen anderen Raum hinein ist in der Regel nicht gewünscht.

Die Betätigungseinheit 6a umfasst darüber hinaus einen Positionssensor 17. Dieser Positionssensor vermag eine Position, Orientierung, Bewegung und / oder Beschleunigung der Betätigungseinheit 6a relativ zu der Versorgungseinheit 2 zu messen. Das Steuergerät 18 der Betätigungseinheit 6a empfängt Signale von dem Positionssensor 17 und prüft, ob die gemessene Position, Orientierung, Bewegung und / oder Beschleunigung der Betätigungseinheit 6a relativ zu der Versorgungseinheit 2 ein vorgegebenes Aktivierungs-Kriterium erfüllt, beispielsweise der Abstand innerhalb einer vorgegebenen maximalen Abstandsschranke liegt. Nur dann, wenn das Aktivierungs-Kriterium erfüllt ist, ermöglicht das Steuergerät 18 der Betätigungseinheit 6a, dass ein Pairing zwischen der Betätigungseinheit 6a und der Versorgungseinheit 2 durchgeführt werden kann.

Bevorzugt wird mindestens einmal nach der Herstellung der Datenverbindung Dv überprüft, ob das Aktivierungs-Kriterium noch erfüllt ist. Bevorzugt wird diese Überprüfung regelmäßig, insbesondere mit fester Frequenz, wiederholt. Falls das Aktivierungs-Kriterium nicht mehr erfüllt ist, so wird bevorzugt die Datenverbindung Dv unterbrochen, beispielsweise durch ein Entkoppeln (Depairing).

In einer Ausgestaltung übermittelt der Sender 8 die gemessene Position, Orientierung, Bewegung und / oder Beschleunigung der Betätigungseinheit 6a an den Empfänger 9. Das Steuergerät 19 der Versorgungseinheit 2 ermöglicht, dass ein Pairing zwischen der Betätigungseinheit 6a und der Versorgungseinheit 2 durchgeführt werden kann.

Die Betätigungseinheiten 6a und 6b und weitere optionale Betätigungseinheiten umfassen jeweils eine eigene Spannungsversorgungseinheit und sind nur dann mit dem stationären Spannungsversorgungsnetz des Krankenhauses verbunden, wenn die Betätigungseinheit 6a, 6b in der Dockingstation 24 positioniert ist. In einer Ausgestaltung umfasst das Versorgungssystem 1 mehr Betätigungseinheiten 6a, 6b als es Dockingstationen 24 gibt. Außerdem befindet sich eine Betätigungseinheit 6a, 6b in der Regel dann, wenn sie für die Betätigung eines Fluid-Versorgungsanschlusses 3, 4, 5 verwendet wird, nicht in der oder einer Dockingstation 24. Um den Verbrauch an elektrischer Energie zu verringern, wird ein Einspar-Mechanismus angewendet, der nachfolgend für die Betätigungseinheit 6a beschrieben ist. Dieser oder ein anderer Einspar-Mechanismus ist bevorzugt auch auf der Betätigungseinheit 6b und der oder jeder weiteren Betätigungseinheit realisiert.

Die Betätigungseinheit 6a befindet sich wahlweise in einem aktivierten Modus, in dem sie eine Betätigung des Betätigungselements 7a, 7b zu erfassen vermag und eine Nachricht mit der Betätigungsinformation abzustrahlen vermag, oder in einem Standby-Modus, in dem der Energieverbrauch im Vergleich zum aktivierten Modus reduziert ist. Die Betätigungseinheit 6a geht von alleine von dem aktivierten Modus in den Standby-Modus über, wenn sie während einer Zeitspanne, die eine vorgegebene Mindestdauer aufweist, nicht benutzt worden ist und insbesondere keine Betätigung eines Betätigungselements 7a, 7b vorgenommen worden ist. Die Betätigungseinheit 6a springt automatisch wieder in den aktivierten Modus, wenn mindestens eines der folgenden Ereignisse eingetreten und detektiert ist:
- Ein Benutzer betätigt ein Betätigungselement 7a, 7b.
- Der Positionssensor 17 detektiert eine Bewegung oder eine Beschleunigung oberhalb einer vorgegebenen unteren Schranke.
- Ein Näherungssensor 20 der Betätigungseinheit 6a erkennt, dass eine Person sich der Betätigungseinheit 6a nähert oder diese gegriffen hat.

### Bezugszeichenliste

| | |
|---|---|
| 1 | Versorgungssystem, umfasst die Versorgungseinheit 2, die Betätigungseinheiten 6a, 6b und optional weitere Betätigungseinheiten |
| 2 | Versorgungseinheit, umfasst den Anschlusskörper 25, den Montagearm 40, den Empfänger 9, die Pairing-Einheit 16 und das Steuergerät 19 |
| 3 | Fluid-Versorgungsanschluss für Sauerstoff an dem Anschlusskörper 25 |
| 4 | Fluid-Versorgungsanschluss für Atemluft mit einer niedrigeren Flussrate an dem Anschlusskörper 25 |
| 5 | Fluid-Versorgungsanschluss für Atemluft mit einer höheren Flussrate an dem Anschlusskörper 25 |
| 6a | Betätigungseinheit des Versorgungssystems 1, umfasst die Betätigungselemente 7a, 7b, den Sender 8, die Pairing-Einheit 15, den Positionssensor 17 und das Steuergerät 18 |
| 6b | weitere Betätigungseinheit des Versorgungssystems 1 |
| 7a, 7b | Betätigungselemente der Betätigungseinheit 6a |
| 8 | Sender der Betätigungseinheit 6a, steht zeitweise in einer drahtlosen Datenverbindung Dv mit dem Empfänger 9 |
| 9 | Empfänger der Versorgungseinheit 2, steht zeitweise in einer drahtlosen Datenverbindung Dv mit dem Sender 8 |
| 10 | seitlicher Außenwand-Abschnitt mit der Aufnahmeeinheit 13 |
| 11 | vorderer Außenwand-Abschnitt mit den Aufnahmeeinheiten 12, 14 |
| 12, 14 | Aufnahmeeinheiten an dem vorderen Außenwand-Abschnitt 11 |
| 13 | Aufnahmeeinheit an dem seitlichen Außenwand-Abschnitt 10 |
| 15 | Pairing-Einheit der Betätigungseinheit 6a |
| 16 | Pairing-Einheit der Versorgungseinheit 2 |
| 17 | Positionssensor der Betätigungseinheit 6a, misst die Position, Orientierung, Bewegung und / oder Beschleunigung der Betätigungseinheit 6a relativ zur Versorgungseinheit 2 |
| 18 | datenverarbeitendes Steuergerät der Betätigungseinheit 6a |
| 19 | datenverarbeitendes Steuergerät der Versorgungseinheit 2 |
| 20 | Näherungssensor der Betätigungseinheit 6a |
| 21 | Vorsprung der Versorgungseinheit 2, an dem der Empfänger 9 angeordnet ist |
| 22 | Verifizierungseinheit der Betätigungseinheit 6a, überprüft, ob ein Benutzer autorisiert ist |
| 24 | Dockingstation an dem vorderen Außenwand-Abschnitt 11 |
| 25 | Anschlusskörper, umfasst die Fluid-Versorgungsanschlüsse 3, 4, 5, den Versorgungsanschluss 27, die Dockingstation 24, die Aufnahmeeinheiten 12, 13, 14, den Vorsprung 21 und die nach oben zeigende Stirnseite 30 |
| 27 | Netzwerkanschluss in Form einer RJ45-Steckdose an dem Anschlusskörper 25 |
| 29 | Montagebereich, der die Stirnseite 30 des Anschlusskörpers 25 mit dem Montagearm 40 verbindet |
| 30 | Stirnseite des Anschlusskörpers 25 |
| 40 | Montagearm, an dem der Anschlusskörper 25 hängt, an einer Decke befestigt oder befestigbar |
| Dv | drahtlose Datenverbindung zwischen dem Sender 8 und dem Empfänger 9 |

## Patentansprüche

1. Versorgungssystem (1) zur Versorgung eines medizinischen Geräts mit mindestens einem Fluid,
wobei das Versorgungssystem eine Versorgungseinheit (2) und eine Betätigungseinheit (6a, 6b) umfasst,
wobei die Versorgungseinheit (2) mindestens einen Fluid-Versorgungsanschluss (3, 4, 5) und einen Empfänger (9) umfasst,
wobei der oder jeder Fluid-Versorgungsanschluss (3, 4, 5) dazu ausgestaltet ist, ein Fluid, insbesondere Sauerstoff und / oder Druckluft, für ein medizinisches Gerät bereitzustellen,
wobei die Betätigungseinheit (6a, 6b)
- mindestens ein von einem Menschen betätigbares Betätigungselement (7a, 7b) sowie einen Sender (8) umfasst und
- als ein tragbares Gerät nach Art einer Fernbedienung ausgestaltet ist,
wobei mindestens zeitweise eine drahtlose Datenverbindung (Dv), insbesondere eine Datenverbindung mittels elektromagnetischer Wellen, zur Nachrichtenübermittlung von dem Sender (8) zu dem Empfänger (9) herstellbar ist,
wobei die Betätigungseinheit (6a, 6b) dazu ausgestaltet ist,
- eine Betätigung des oder eines Betätigungselements (7a, 7b) zu erfassen und
- abhängig von der erfassten Betätigung eine Nachricht mit einer die Betätigung kennzeichnenden Betätigungsinformation zu erzeugen und über die Datenverbindung (Dv) an den Empfänger (9) zu übermitteln,
wobei die Versorgungseinheit (2) dazu ausgestaltet ist, als Reaktion auf den Empfang dieser Nachricht mit der Betätigungsinformation einen Parameter der Versorgungseinheit (2) zu verändern,
wobei dieser Parameter die Bereitstellung von Fluid durch den oder mindestens einen Fluid-Versorgungsanschluss (3, 4, 5) beeinflusst.

2. Versorgungssystem (1) nach Anspruch 1,
**dadurch gekennzeichnet, dass**
die Versorgungseinheit (2) eine erste Aufnahmeeinheit (12) umfasst,
welche dazu ausgestaltet ist, die oder eine Betätigungseinheit (6a, 6b) aufzunehmen und lösbar zu halten,
wobei die Betätigungseinheit (6a, 6b) in die erste Aufnahmeeinheit (12) einsetzbar und wieder aus der ersten Aufnahmeeinheit (12) entnehmbar ist.

3. Versorgungssystem (1) nach Anspruch 2,
**dadurch gekennzeichnet, dass**
das Versorgungssystem mindestens eine zweite Aufnahmeeinheit (13, 14) umfasst,
wobei die oder jede zweite Aufnahmeeinheit (13,14) räumlich von der ersten Aufnahmeeinheit (12) beabstandet ist und ebenfalls dazu ausgestaltet ist, die Betätigungseinheit (6a, 6b) aufzunehmen und zu halten,
wobei die oder eine Betätigungseinheit (6a, 6b) wahlweise in die erste Aufnahmeeinheit (12) oder in die oder eine zweite Aufnahmeeinheit (13,14) einsetzbar und wieder aus dieser Aufnahmeeinheit (12, 13, 14) entnehmbar ist.

4. Versorgungssystem (1) nach einem der vorhergehenden Ansprüche,
**dadurch gekennzeichnet, dass**
das Versorgungssystem (1) mindestens zwei Betätigungseinheiten (6a, 6b) umfasst und
das Versorgungssystem (1) so ausgestaltet ist, dass dann, wenn eine Datenverbindung (Dv) zur Nachrichtenübermittlung von dem Sender (8) einer Betätigungseinheit (6a) zu dem Empfänger (9) hergestellt ist,
das Herstellen einer Datenverbindung (Dv) zur Nachrichtenübermittlung von dem Sender einer anderen Betätigungseinheit (6b) zu dem Empfänger (9) gesperrt ist.

5. Versorgungssystem (1) nach Anspruch 4,
**dadurch gekennzeichnet, dass**
jede Betätigungseinheit (6a, 6b) jeweils eine erste Pairing-Einheit (15) umfasst und
die Versorgungseinheit (2) eine zweite Pairing-Einheit (16) umfasst,
wobei die beiden Pairing-Einheiten (15, 16) dazu ausgestaltet sind, ein Pairing zwischen dieser Betätigungseinheit (6a) und der Versorgungseinheit (2) durchzuführen.

6. Versorgungssystem (1) nach einem der vorhergehenden Ansprüche,
**dadurch gekennzeichnet, dass**
nach Herstellen einer Datenverbindung (Dv) zwischen dem oder einem Sender (8) und dem Empfänger (9) eine Nachricht vom Empfänger (9) zum Sender (8) übertragbar ist,
wobei die Versorgungseinheit (2) einen Datenspeicher aufweist, in dem ein rechnerauswertbarer Schlüssel abgespeichert ist,
wobei der Empfänger (9) dazu ausgestaltet ist, eine Nachricht mit dem Schlüssel an den Sender (8) zu übermitteln, und
wobei der Sender (8) dazu ausgestaltet ist, automatisch
- unter Verwendung des empfangenen Schlüssels die oder jede Nachricht mit einer Betätigungsinformation zu verschlüsseln und
- die verschlüsselte Nachricht über die Datenverbindung (Dv) an den Empfänger (9) zu übermitteln.

7. Versorgungssystem (1) nach einem der vorhergehenden Ansprüche,
**dadurch gekennzeichnet, dass**
das Versorgungssystem (1) einen Positionssensor (17) umfasst,
welcher dazu ausgestaltet ist, eine Position, Orientierung, Bewegung und / oder Beschleunigung der oder einer Betätigungseinheit (6a, 6b) relativ zu der Versorgungseinheit (2) und / oder eine Entfernung zwischen der Betätigungseinheit (6a, 6b) und der Versorgungseinheit (2) zu detektieren,
wobei das Versorgungssystem (1) dazu ausgestaltet ist, die Herstellung einer Datenverbindung (Dv) zwischen dem Sender (8) einer Betätigungseinheit (6a, 6b) und dem Empfänger (9) abhängig von einem Signal des Positionssensors (17) zu ermöglichen oder zu unterbinden.

8. Anordnung umfassend
- ein medizinisches Gerät, insbesondere ein Beatmungsgerät oder ein Anästhesiegerät oder ein Patienten-Monitor, und
- ein Versorgungssystem (1) nach einem der vorhergehenden Ansprüche,
wobei das medizinische Gerät mit dem oder mindestens einem Fluid-Versorgungsanschluss (3, 4, 5) des Versorgungssystems (1) verbunden oder verbindbar ist.

9. Verwendung eines Versorgungssystems (1) nach einem der Ansprüche 1 bis 7
zur Versorgung eines medizinischen Geräts, insbesondere eines Beatmungsgeräts oder eines Anästhesiegeräts oder eines Patienten-Monitors,
mit mindestens einem Fluid, insbesondere mit Atemluft und / oder Sauerstoff.

10. Verfahren zum Versorgen eines medizinischen Geräts mit mindestens einem Fluid
unter Verwendung eines Versorgungssystems (1), welches eine Versorgungseinheit (2) und eine Betätigungseinheit (6a, 6b) umfasst,
wobei die Versorgungseinheit (2) mindestens einen Fluid-Versorgungsanschluss (3, 4, 5) und einen Empfänger (9) umfasst,
wobei die Betätigungseinheit (6a, 6b)
- mindestens ein von einem Menschen betätigbares Betätigungselement (7a, 7b) sowie einen Sender (8) umfasst und
- als ein tragbares Gerät nach Art einer Fernbedienung ausgestaltet ist,
wobei der oder jeder Fluid-Versorgungsanschluss (3, 4, 5) dazu ausgestaltet ist, ein Fluid, insbesondere Sauerstoff und / oder Druckluft, für ein medizinisches Gerät bereitzustellen, und
wobei das Verfahren die automatisch durchgeführten Schritte umfasst, dass
- die Betätigungseinheit (6a, 6b) eine Betätigung des oder eines Betätigungselements (7a, 7b) erfasst,
- die Betätigungseinheit (6a, 6b) abhängig von der erfassten Betätigung eine Nachricht mit einer die Betätigung kennzeichnenden Betätigungsinformation erzeugt,
- eine drahtlose Datenverbindung (Dv), insbesondere mittels elektromagnetischer Wellen, zur Nachrichtenübermittlung von dem Sender (8) zu dem Empfänger (9) hergestellt wird,
- die erzeugte Nachricht über die hergestellte Datenverbindung (Dv) von dem Sender (8) zu dem Empfänger (9) übermittelt wird und
- als Reaktion auf den Empfang der Betätigungsinformation ein Parameter der Versorgungseinheit (2) verändert wird,
wobei dieser Parameter die Bereitstellung von Fluid durch den oder mindestens einen Fluid-Versorgungsanschluss (3, 4, 5) beeinflusst.

11. Verfahren nach Anspruch 10,
**dadurch gekennzeichnet, dass**
die drahtlose Datenverbindung (Dv) zur Nachrichtenübermittlung von dem Sender (8) zu dem Empfänger (9) nur dann hergestellt wird, wenn ein vorgegebenes Verbindungs-Kriterium erfüllt ist,
wobei das Verbindungs-Kriterium mindestens dann erfüllt ist, wenn
- die durch einen Positionssensor (17) gemessene Position, Orientierung, Bewegung und / oder Beschleunigung der Betätigungseinheit (6a, 6b) relativ zu der Versorgungseinheit (2) ein vorgegebenes Aktivierungs-Kriterium erfüllt und / oder
- der Abstand zwischen der Betätigungseinheit (6a, 6b) und der Versorgungseinheit (2) kleiner oder gleich einem vorgegebenen maximalen Abstand ist und / oder
- durch eine Verifizierungseinheit (22) verifiziert ist, dass die erfasste Betätigung des Betätigungselements (7a, 7b) von einem autorisierten Benutzer durchgeführt worden ist, und / oder
- aktuell keine Datenverbindung von dem Sender einer anderen Betätigungseinheit zu dem Empfänger (9) hergestellt ist.

## Claims

1. Supply system (1) for supplying a medical appliance with at least one fluid,
wherein the supply system comprises a supply unit (2) and an actuating unit (6a, 6b),
wherein the supply unit (2) comprises at least one fluid supply connection point (3, 4, 5) and one receiver (9),
wherein each fluid supply connection point (3, 4, 5) is configured to provide a fluid, in particular oxygen and / or compressed air, for a medical appliance,
wherein the actuating unit (6a, 6b)
- comprises at least one actuating element (7a, 7b), which can be actuated by a person, and a sender (8) and
- is configured as a portable appliance in the style of a remote control,
wherein a wireless data connection (Dv), in particular a data connection by means of electromagnetic waves, can be established, at least temporarily, for message transmission from the sender (8) to the receiver (9),
wherein the actuating unit (6a, 6b) is configured
- to detect an actuation of the or an actuating element (7a, 7b) and
- depending on the detected actuation, to generate a message containing actuation information that characterizes the actuation and to transmit it to the receiver (9) via the data connection (Dv),
wherein the supply unit (2) is configured to change a parameter of the supply unit (2) in response to receiving this message containing the actuation information,
wherein this parameter affects the provision of fluid through the or at least one fluid supply connection point (3, 4, 5).

2. Supply system (1) according to claim 1,
**characterized in that**
the supply unit (2) comprises a first receiving unit (12)
which is configured to receive the or an actuating unit (6a, 6b) and detachably hold it,
it being possible to insert the actuating unit (6a, 6b) into the first receiving unit (12) and remove it again from the first receiving unit (12).

3. Supply system (1) according to claim 2,
**characterized in that**
the supply system comprises at least one second receiving unit (13, 14),
the or each second receiving unit (13,14) being spatially separated from the first receiving unit (12) and also being configured to receive and hold the actuating unit (6a, 6b),
it being possible, optionally, to insert the or an actuating unit (6a, 6b) into the first receiving unit (12) or into the or a second receiving unit (13,14) and to remove it again from this receiving unit (12, 13, 14).

4. Supply system (1) according to any of the preceding claims,
**characterized in that**
the supply system (1) comprises at least two actuating units (6a, 6b) and
the supply system (1) is configured such that if a data connection (Dv) for message transmission from the sender (8) of an actuating unit (6a) to the receiver (9) is established,
the establishment of a data connection (Dv) for message transmission from the sender of another actuating unit (6b) to the receiver (9) is blocked.

5. Supply system (1) according to claim 4,
**characterized in that**
each actuating unit (6a, 6b) comprises a first pairing unit (15) and
the supply unit (2) comprises a second pairing unit (16),
the two pairing units (15, 16) being configured to perform a pairing between this actuating unit (6a) and the supply unit (2).

6. Supply system (1) according to any of the preceding claims,
**characterized in that**
after a data connection (Dv) is established between the or a sender (8) and the receiver (9), a message can be transferred from the receiver (9) to the sender (8),
the supply unit (2) having a data storage device in which a computer-evaluable key is stored,
the receiver (9) being configured to transmit a message containing the key to the sender (8), and
the sender (8) being configured to automatically
- encrypt the or each message containing actuation information using the received key and
- transmit the encrypted message to the receiver (9) via the data connection (Dv).

7. Supply system (1) according to any of the preceding claims,
**characterized in that**
the supply system (1) comprises a position sensor (17)
which is configured to detect a position, orientation, movement and / or acceleration of the or an actuating unit (6a, 6b) relative to the supply unit (2) and / or a distance between the actuating unit (6a, 6b) and the supply unit (2),
the supply system (1) being configured to enable or prevent the establishment of a data connection (Dv) between the sender (8) of an actuating unit (6a, 6b) and the receiver (9) depending on a signal from the position sensor (17).

8. Arrangement comprising
- a medical appliance, in particular a ventilator or an anesthetic machine or a patient monitor, and
- a supply system (1) according to any of the preceding claims,
wherein the medical appliance is or can be connected to the or at least one fluid supply connection point (3, 4, 5) of the supply system (1).

9. Use of a supply system (1) according to any of claims 1 to 7
for supplying a medical appliance, in particular a ventilator or an anesthetic machine or a patient monitor,
with at least one fluid, in particular with breathing air and / or oxygen.

10. Method for supplying a medical appliance with at least one fluid
using a supply system (1) comprising a supply unit (2) and an actuating unit (6a, 6b),
wherein the supply unit (2) comprises at least one fluid supply connection point (3, 4, 5) and one receiver (9),
wherein the actuating unit (6a, 6b)
- comprises at least one actuating element (7a, 7b), which can be actuated by a person, and a sender (8) and
- is configured as a portable appliance in the style of a remote control,
wherein the or each fluid supply connection point (3, 4, 5) is configured to provide a fluid, in particular oxygen and / or compressed air, for a medical appliance, and
wherein the method comprises the following automatically performed steps:
- the actuating unit (6a, 6b) detects an actuation of the or an actuating element (7a, 7b),
- the actuating unit (6a, 6b) generates a message containing actuation information that characterizes the actuation depending on the detected actuation,
- a wireless data connection (Dv), in particular by means of electromagnetic waves, is established for message transmission from the sender (8) to the receiver (9),
- the generated message is transmitted via the established data connection (Dv) from the sender (8) to the receiver (9) and
- in response to the receipt of the actuation information, a parameter of the supply unit (2) is changed,
wherein this parameter affects the provision of fluid through the or at least one fluid supply connection point (3, 4, 5).

11. Method according to claim 10,
**characterized in that**
the wireless data connection (Dv) for message transmission from the sender (8) to the receiver (9) is only established if a predefined connection criterion is fulfilled,
the connection criterion being fulfilled at least if
- the position, orientation, movement and / or acceleration of the actuating unit (6a, 6b) relative to the supply unit (2), as measured by a position sensor (17), fulfills a predefined activation criterion and / or
- the distance between the actuating unit (6a, 6b) and the supply unit (2) is less than or equal to a predefined maximum distance and / or
- it is verified by a verification unit (22) that the detected actuation of the actuating element (7a, 7b) was performed by an authorized user, and / or
- currently, no data connection is established between the sender of another actuating unit and the receiver (9).

## Revendications

1. Système d'alimentation (1) permettant d'alimenter un appareil médical avec au moins un fluide,
dans lequel le système d'alimentation comprend une unité d'alimentation (2) et une unité d'actionnement (6a, 6b),
dans lequel l'unité d'alimentation (2) comprend au moins un raccord d'alimentation en fluide (3, 4, 5) et un récepteur (9),
dans lequel le raccord d'alimentation en fluide ou chaque raccord d'alimentation en fluide (3, 4, 5) est conçu pour fournir un fluide, en particulier de l'oxygène et/ou de l'air comprimé, à un appareil médical,
dans lequel l'unité d'actionnement (6a, 6b)
- comprend au moins un élément d'actionnement (7a, 7b) pouvant être actionné par un être humain ainsi qu'un émetteur (8) et
- est conçu sous la forme d'un appareil portable de type télécommande,
dans lequel une connexion de données (Dv) sans fil, en particulier une connexion de données par le biais d'ondes électromagnétiques, peut être établie au moins temporairement pour la transmission de messages de l'émetteur (8) vers le récepteur (9),
dans lequel l'unité d'actionnement (6a, 6b) est configurée pour
- détecter un actionnement de l'élément d'actionnement ou d'un élément d'actionnement (7a, 7b) et
- générer, en fonction de l'actionnement détecté, un message comportant des informations d'actionnement caractérisant l'actionnement et le transmettre au récepteur (9) par l'intermédiaire de la connexion de données (Dv),
dans lequel l'unité d'alimentation (2) est configurée pour modifier un paramètre de l'unité d'alimentation (2) en réponse à la réception de ce message comportant les informations d'actionnement,
dans lequel ce paramètre influence la fourniture de fluide par le raccord d'alimentation en fluide ou au moins un raccord d'alimentation en fluide (3, 4, 5).

2. Système d'alimentation (1) selon la revendication 1,
**caractérisé en ce que**
l'unité d'alimentation (2) comprend une première unité de réception (12),
laquelle est conçue pour recevoir et maintenir de manière amovible l'unité d'actionnement ou une unité d'actionnement (6a, 6b),
dans lequel l'unité d'actionnement (6a, 6b) peut être insérée dans la première unité de réception (12) et peut être à nouveau retirée de la première unité de réception (12).

3. Système d'alimentation (1) selon la revendication 2,
**caractérisé en ce que**
le système d'alimentation comprend au moins une seconde unité de réception (13, 14),
dans lequel la seconde unité de réception ou chaque seconde unité de réception (13, 14) est espacée de la première unité de réception (12) et est également conçue pour recevoir et maintenir l'unité d'actionnement (6a, 6b),
dans lequel l'unité d'actionnement ou une unité d'actionnement (6a, 6b) peut être insérée au choix dans la première unité de réception (12) ou dans la seconde unité de réception ou une seconde unité de réception (13, 14), et peut à nouveau être retirée de cette unité de réception (12, 13, 14).

4. Système d'alimentation (1) selon l'une des revendications précédentes,
**caractérisé en ce que**
le système d'alimentation (1) comprend au moins deux unités d'actionnement (6a, 6b) et
le système d'alimentation (1) est configuré de telle sorte que, lorsqu'une connexion de données (Dv) est établie pour la transmission de messages de l'émetteur (8) d'une unité d'actionnement (6a) vers le récepteur (9),
l'établissement d'une connexion de données (Dv) pour la transmission de messages depuis l'émetteur d'une autre unité d'actionnement (6b) vers le récepteur (9) est bloqué.

5. Système d'alimentation (1) selon la revendication 4,
**caractérisé en ce que**
chaque unité d'actionnement (6a, 6b) comprend respectivement une première unité d'appariement (15) et
l'unité d'alimentation (2) comprend une seconde unité d'appariement (16),
dans lequel les deux unités d'appariement (15, 16) sont conçues pour exécuter un appariement entre cette unité d'actionnement (6a) et l'unité d'alimentation (2).

6. Système d'alimentation (1) selon l'une des revendications précédentes,
**caractérisé en ce que**
après l'établissement d'une connexion de données (Dv) entre l'émetteur ou un émetteur (8) et le récepteur (9), un message peut être transmis du récepteur (9) à l'émetteur (8),
dans lequel l'unité d'alimentation (2) présente une mémoire de données dans laquelle est enregistrée une clé exploitable par ordinateur,
dans lequel le récepteur (9) est configuré pour transmettre un message avec la clé à l'émetteur (8), et
dans lequel l'émetteur (8) est configuré pour
- crypter automatiquement, à l'aide de la clé reçue, le message ou chaque message comportant des informations d'actionnement et
- transmettre automatiquement le message crypté au récepteur (9) par l'intermédiaire de la connexion de données (Dv).

7. Système d'alimentation (1) selon l'une des revendications précédentes,
**caractérisé en ce que**
le système d'alimentation (1) comprend un capteur de position (17),
lequel est configuré pour détecter une position, une orientation, un mouvement et/ou une accélération de l'unité d'actionnement ou d'une unité d'actionnement (6a, 6b) par rapport à l'unité d'alimentation (2) et/ou pour détecter une distance entre l'unité d'actionnement (6a, 6b) et l'unité d'alimentation (2),
dans lequel le système d'alimentation (1) est configuré pour permettre ou empêcher l'établissement d'une connexion de données (Dv) entre l'émetteur (8) d'une unité d'actionnement (6a, 6b) et le récepteur (9) en fonction d'un signal du capteur de position (17).

8. Agencement comprenant
- un appareil médical, en particulier un respirateur ou un appareil d'anesthésie ou un moniteur patient, et
- un système d'alimentation (1) selon l'une des revendications précédentes,
dans lequel l'appareil médical est connecté ou peut être connecté au raccord d'alimentation en fluide ou à au moins un raccord d'alimentation en fluide (3, 4, 5) du système d'alimentation (1).

9. Utilisation d'un système d'alimentation (1) selon l'une des revendications 1 à 7
pour l'alimentation d'un appareil médical, en particulier d'un respirateur ou d'un appareil d'anesthésie ou d'un moniteur patient,
avec au moins un fluide, en particulier avec de l'air respirable et/ou de l'oxygène.

10. Procédé permettant d'alimenter un appareil médical avec au moins un fluide
à l'aide d'un système d'alimentation (1) qui comprend une unité d'alimentation (2) et une unité d'actionnement (6a, 6b),
dans lequel l'unité d'alimentation (2) comprend au moins un raccord d'alimentation en fluide (3, 4, 5) et un récepteur (9),
dans lequel l'unité d'actionnement (6a, 6b)
- comprend au moins un élément d'actionnement (7a, 7b) pouvant être actionné par un être humain ainsi qu'un émetteur (8) et
- est conçu sous la forme d'un appareil portable de type télécommande,
dans lequel le raccord d'alimentation en fluide ou chaque raccord d'alimentation en fluide (3, 4, 5) est conçu pour fournir un fluide, en particulier de l'oxygène et/ou de l'air comprimé, à un appareil médical, et
dans lequel le procédé comprend les étapes exécutées automatiquement selon lesquelles
- l'unité d'actionnement (6a, 6b) détecte un actionnement de l'élément d'actionnement ou d'un élément d'actionnement (7a, 7b),
- l'unité d'actionnement (6a, 6b) génère, en fonction de l'actionnement détecté, un message comportant des informations d'actionnement caractérisant l'actionnement,
- une connexion de données (Dv) sans fil est établie, en particulier par le biais d'ondes électromagnétiques, pour la transmission de messages de l'émetteur (8) vers le récepteur (9),
- le message généré est transmis de l'émetteur (8) au récepteur (9) par l'intermédiaire de la connexion de données (Dv) établie et
- un paramètre de l'unité d'alimentation (2) est modifié en réponse à la réception des informations d'actionnement,
dans lequel ce paramètre influence la fourniture de fluide par le raccord d'alimentation en fluide ou au moins un raccord d'alimentation en fluide (3, 4, 5).

11. Procédé selon la revendication 10,
**caractérisé en ce que**
la connexion de données (Dv) sans fil pour la transmission de messages de l'émetteur (8) vers le récepteur (9) n'est établie que lorsqu'un critère de connexion prédéfini est satisfait,
dans lequel le critère de connexion est satisfait au moins lorsque
- la position, l'orientation, le mouvement et/ou l'accélération de l'unité d'actionnement (6a, 6b) par rapport à l'unité d'alimentation (2), mesurés par un capteur de position (17), satisfont à un critère d'activation prédéfini et/ou
- la distance entre l'unité d'actionnement (6a, 6b) et l'unité d'alimentation (2) est inférieure ou égale à une distance maximale prédéfinie et/ou
- une unité de vérification (22) vérifie que l'actionnement détecté de l'élément d'actionnement (7a, 7b) a été exécuté par un utilisateur autorisé, et/ou
- aucune connexion de données n'est actuellement établie entre l'émetteur d'une autre unité d'actionnement et le récepteur (9).
